# EUROPEAN PATENT APPLICATION

(11) **EP 2 959 823 A1**
(43) Date of publication of application: **30.12.2015**
(21) Application number: 14814490.0
(22) Date of filing: 08.05.2014
(51) Int. Cl.: A61B 1/00, G02B 23/24

(54) **ENDOSCOPE**

(30) Priority: 19.06.2013 JP 2013128837
(71) Applicant: OLYMPUS CORPORATION, Shibuya-ku Tokyo 151-0072 (JP)
(72) Inventor: SHIMADA Naoya, Tokyo 151-0072 (JP)
(74) Representative: Schicker, Silvia
(86) International application number: PCT/JP2014/062334
(87) International publication number: WO 2014/203641

(57) **Abstract**

An endoscope including: a tubular flexible member which has elasticity and configures an outermost layer of a bending portion; a bending portion fixing rigid portion at which an end portion of the tubular flexible member is arranged; and a bending portion end fixing portion for fixing the end portion of the tubular flexible member arranged at the bending portion fixing rigid portion, in which the bending portion includes, at an end portion in a longitudinal axis thereof, a bending portion end small-diameter portion, an outer diameter dimension of which is set to be small, and an outer diameter of a covering portion which configures the bending portion end fixing portion is smaller than an outer diameter of a bending portion fixing rigid portion-side end portion and an outer diameter of an end portion of a middle tube portion of the tubular flexible member, the bending portion fixing rigid portion-side end portion and the end portion of the middle tube portion being arranged so as to be opposed to each other across the bending portion end small-diameter portion.

## Description

### Technical Field

The present invention relates to an endoscope which includes at an insertion portion thereof a bending portion and which is used by being passed through a through hole of a guiding pipe or the like.

### Background Art

Endoscopes have been used in medical fields, industrial fields, and the like. Such endoscopes include a type in which a bending portion is provided at the distal end side of an elongated and flexible insertion portion. The endoscope including a bending portion enables an observation in a wide range to be performed by changing a field-of-view direction of an observation optical system provided at a distal end portion of an insertion portion through the bending of the bending portion.

The bending portion is mainly configured by a bending piece group, a bending rubber, and bending wires.

The bending piece group is configured to pivotably coupling a plurality of bending pieces in a longitudinal axis direction, to allow the bending portion to be bent in two directions, i.e., up and down directions, or in four directions, i.e., up, down, right and left directions, for example. The distal end of each of the bending wires is fixed to a predetermined position of a distal end bending piece located at the distal-most end of the bending piece group.

The bending rubber is a covering member, and configures an outer layer of the bending portion by covering an outer circumference of the bending piece group which is formed to be elongate. The distal end portion of the bending rubber is fixed to an outer circumference of a distal end constituting member which constitutes the distal end portion of the insertion portion. The proximal end portion of the bending rubber is fixed to an outer circumference of a coupling cap that couples a flexible tube portion and a proximal end bending piece located at the proximal-most end of the bending piece group.

In general, the end portions of the bending rubber are tightly fixed while maintaining watertightness through thread-winding adhesion including a thread-winding operation and an adhesion operation. The thread-winding operation is an operation for tightly binding thread around the outer circumferential surface of the bending rubber to flatten out the bending rubber in the inner circumferential direction, and pressing and fixing the inner circumferential surface of the bending rubber against the outer circumferential surface of the distal end constituting member, or the like.

The adhesion operation is an operation for ensuring watertightness between a distal end rigid member and the bending rubber by applying adhesive to the tightly-bound part and the part around thereof after completion of the thread-winding operation, and improving insertion performance by smoothing the external appearance of the applied adhesive.

If the endoscope is a baby endoscope to be inserted into a through hole of a treatment instrument channel of a mother endoscope as shown in Japanese Patent Application Laid-Open Publication No. 2012-95719, for example, the insertion portion of the baby endoscope is inserted into the treatment instrument channel, and thereafter led out to the outside from the mother endoscope through a treatment instrument raising stand.

Therefore, the insertion portion of the baby endoscope contacts the channel inner surface when being inserted into or extracted from the treatment instrument channel, and contacts the treatment instrument raising stand when being led out to the outside from the mother endoscope or introduced from outside into the mother endoscope.

Such a contact between the thread-wound adhering portion and the channel inner surface or the treatment instrument raising stand may cause problems such as peeling-off of the adhering portion, a cutoff of the bound thread, and the like.

In addition, friction occurred between the bending rubber and the channel inner surface or friction occurred between the bending rubber and the treatment instrument raising stand may cause slackening on the surface of the bending rubber. Then, such slackening is deformed into folds, which may lead to a local appearance of a bulged portion with a large diameter. In addition, the bulged portion is rubbed, which may create a hole on the bending rubber.

Note that the above-described problems do not limitedly occur in the baby endoscope which is configured to be insertable into and extractable from the treatment instrument channel. For example, the above-described problems may occur in other endoscopes, when the endoscopes are introduced into a body through a through hole of a guiding pipe such as a guide tube.

Japanese Utility Model Application Laid-Open Publication No. 5-91602 discloses the endoscope configured such that the end portion of the outer cover layer at the angle portion provided at the insertion portion of the endoscope can be fixed by winding thread and filling an adhesive which does not bulge. In this endoscope, a technique is used in which a rigid ring is slid to be arranged at the thread-wound portion, and thereafter adhesive is applied into a through hole so as not to bulge from the outer surface, to thereby fix the rigid ring.

According to the above-described technique, the adhesive is applied into the through hole so as not to bulge from the outer surface, to protect the thread-wound portion with the rigid ring, thereby solving the problems which may occur at the adhering portion and the thread-wound portion.

However, in the technique disclosed in the Japanese Utility Model Application Laid-Open Publication No. 5-91602, it is difficult to prevent such a problem that the outer diameter size of the surface of the bending rubber is locally increased to create a bulged portion due to friction. Therefore, the bulged portion is rubbed, which may create a hole on the bending rubber. When a hole is created, the operation for removing the adhered and fixed rigid ring is complicated, which degrades the repairability of the endoscope.

The present invention has been achieved in view of the above-described circumstances, and an object of the present invention is to provide an endoscope which prevents a problem caused by a thread-wound adhering portion being rubbed and a problem that a hole is created by a bulged portion appeared by friction being rubbed.

### Disclosure of Invention

### Means for Solving the Problem

An endoscope according to one aspect of the present invention is an endoscope including: a tubular flexible member which has elasticity and configures an outermost layer of a bending portion; a bending portion fixing rigid portion at which an end portion of the tubular flexible member is arranged; and a bending portion end fixing portion for fixing the end portion of the tubular flexible member arranged at the bending portion fixing rigid portion, wherein the bending portion includes, at an end portion in a longitudinal axis thereof, a bending portion end small-diameter portion, an outer diameter dimension of which is set to be small, and an outer diameter of a covering portion which configures the bending portion end fixing portion is smaller than an outer diameter of the bending portion fixing rigid portion-side end portion and an outer diameter of an end portion of a middle tube portion of the tubular flexible member, the bending portion fixing rigid portion-side end portion and the end portion of the middle tube portion being arranged so as to be opposed to each other across the bending portion end small-diameter portion.

### Brief Description of the Drawings

FIG. 1 illustrates a cholangioscope system as one example of a mother-baby endoscope configured by an endoscope and a side-view endoscope according to the present invention.
FIG. 2 illustrates a cholangioscope in the cholangioscope system.
FIG. 3 illustrates the cholangioscope led out from a distal end portion of the side-view endoscope into the duodenum.
FIG. 4 illustrates an exemplary configuration of a bending portion of the cholangioscope.
FIG. 5 illustrates a configuration of a distal end constituting portion and a fixing structure between the distal end constituting portion and a distal end portion of the bending portion.
FIG. 6 illustrates a connecting structure between the bending portion and a flexible tube portion and a fixing structure between a proximal end portion of the bending portion and a connection tube.
FIG. 7 illustrates a state where a distal end portion side of the insertion portion passes through a channel tube.
FIG. 8 illustrates a bulged portion which appears by friction between a distal end portion of a treatment instrument raising stand and a surface of a bending rubber.
FIG. 9A illustrates another configuration of an end portion of the bending rubber.
FIG. 9B illustrates a fixing structure between a bending portion including the end portion of the bending rubber having the other configuration and the distal end constituting portion.
FIG. 10 is a cross-sectional view taken along Y10-Y10 line in FIG. 1, and illustrates an exemplary configuration of a channel tube of a second endoscope and a first endoscope.
FIG. 11 illustrates a configuration of a guide-wire-dedicated channel provided in the insertion portion.

### Best Mode for Carrying Out the Invention

Hereinafter, embodiments of the present invention will be described with reference to drawings.

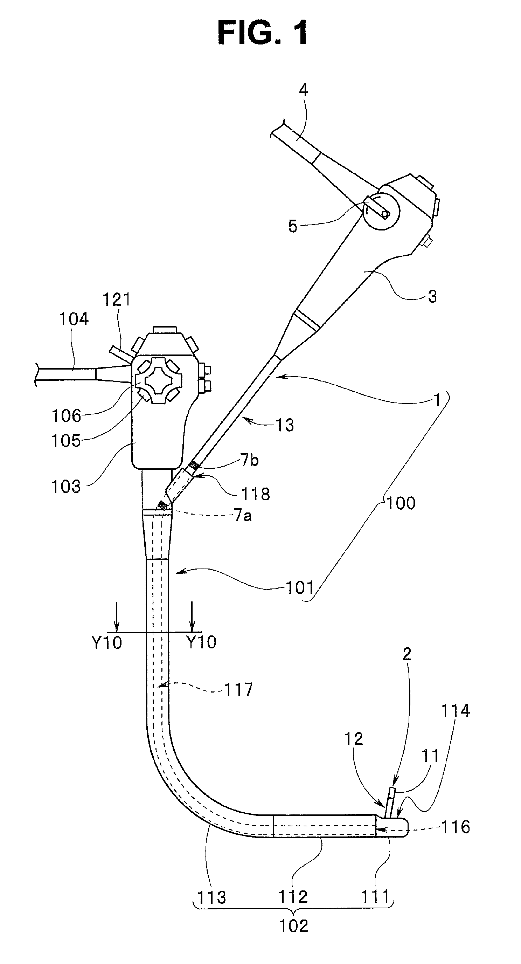

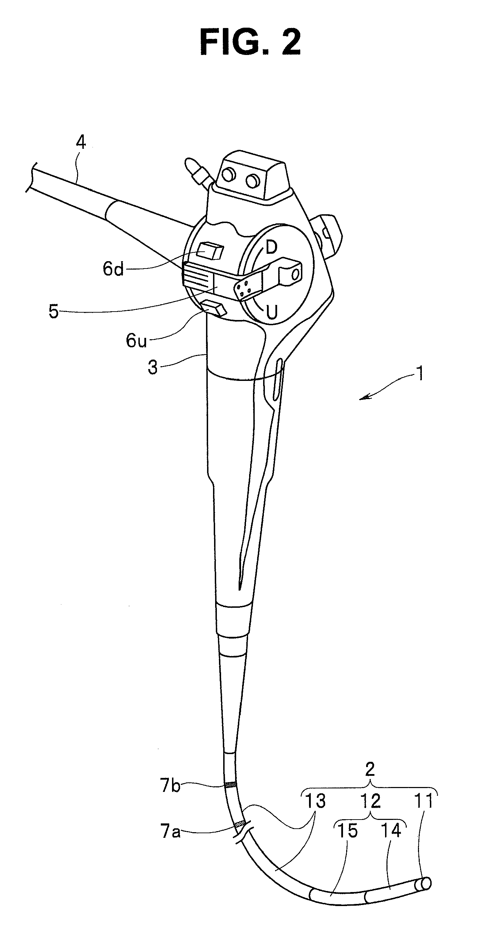

A mother-baby endoscope system shown in FIG. 1 is a cholangioscope system 100. The cholangioscope system 100 includes a cholangioscope 1 (see FIG. 2) and a side-view endoscope 101.

In order to discriminate the configuration of the cholangioscope 1 from the configuration of the side-view endoscope 101, the cholangioscope 1 is also referred to as a first endoscope 1 and the side-view endoscope 101 is also referred to as a second endoscope 101.

In addition, when the names of members or portions related to the configuration of the endoscope 1 are the same as those related to the configuration of the side-view endoscope 101, the term "second" is added to the names of the members or portions of the second endoscope 101, to discriminate them from the members or portions of the same names which are related to the configuration of the first endoscope 1.

As shown in FIG. 1, the side-view endoscope 101 includes a second insertion portion 102, a second operation portion 103 provided continuously with the second insertion portion 102, and a second universal cord 104 extended from the second operation portion 103.

The second universal cord 104 includes at an extension end thereof a second endoscope connector, not shown. The second endoscope connector is connectable to external apparatuses such as an image processing apparatus, light source apparatus, and the like, not shown, which are external apparatuses of the second endoscope 101.

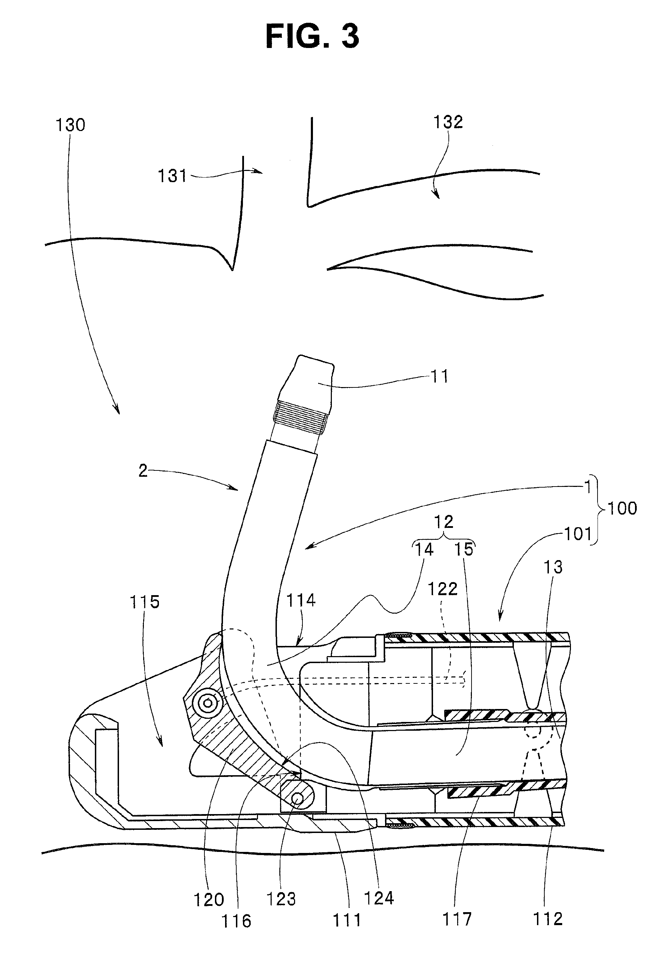

The second insertion portion 102 is inserted through the mouth and advanced to the duodenum (see the reference sign 130 in FIG. 3). The second insertion portion 102 includes in the following order from the distal end side, a rigid second distal end portion 111, a second bending portion 112, and the second flexible tube portion 113, which are provided in a linked manner.

The second bending portion 112 is configured to be bent in four directions, i.e., up, down, right and left directions, for example. The second operation portion 103 is provided with an up/down bending operation knob 105 and a right/left bending operation knob 106. The second bending portion 112 is configured to be bent in up, down, right, and left directions by the knob 105 or 106 being appropriately operated to allow the bending operation wires (not shown) corresponding to the bending directions to be pulled or relaxed.

As shown in FIGS. 1 and 3, a cutout 115 is formed on a part of an outer circumferential side surface 114 of the second distal end portion 111. Inside the cutout 115, a treatment instrument raising stand 120 is provided. An image pickup window (not shown) of an image pickup optical system which constitutes an observation optical system not shown and an illumination window (not shown) of an illumination optical system are provided on the outer circumferential side surface 114. The second distal end portion 111 incorporates inside thereof an image pickup unit (not shown) which constitutes an image pickup optical system.

Inside the cutout 115, a distal end opening 116 is provided. The distal end opening 116 communicates with a through-hole distal end opening of a channel tube 117 which constitutes a treatment instrument channel. The channel tube 117 is a flexible tube body. The channel tube 117 is inserted and disposed in the second insertion portion 102. A through-hole proximal end opening of the channel tube 117 communicates with a treatment instrument insertion port 118 provided at the operation portion 103.

The treatment instrument raising stand 120 is a known device for changing the leading-out direction of the insertion portion 2 to be described later of the first endoscope 1, which is inserted through the channel tube 117 and led out from the distal end opening 116. The treatment instrument raising stand 120 is configured to pivot around a pivot shaft 123 by a treatment instrument raising stand operation lever 121 provided on the second operation portion 103 being operated to allow a raising stand operation wire 122 to be pulled or relaxed.

As shown in FIG. 2, the cholangioscope 1 includes an insertion portion 2, an operation portion 3 provided continuously with the insertion portion 2, a universal cord 4 extended from the operation portion 3. A cholangioscope connector (not shown) is provided at an extension end of the universal cord 4. The cholangioscope connector is configured to be connectable to external apparatuses of the cholangioscope 1, such as an image processing apparatus, a light source apparatus, etc., not shown.

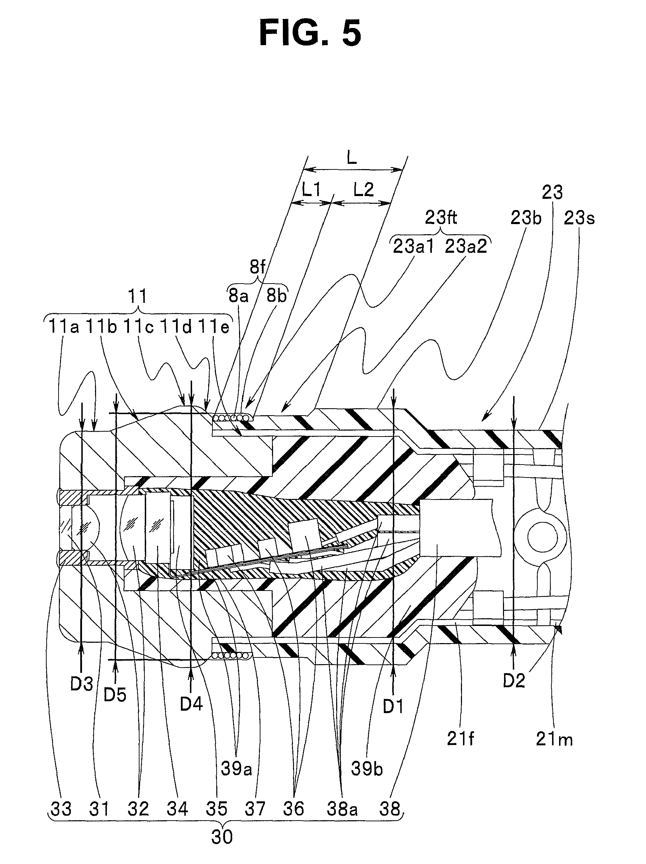

The cholangioscope 1 is a front-view endoscope. An image pickup window (reference sign 31 in FIG. 5) which constitutes an image pickup optical system and an illumination window (not shown) which constitutes an illumination optical system are provided on a distal end surface of a distal end constituting portion 11 of the cholangioscope 1.

The insertion portion 2 is configured to have an outer diameter which can be insertable into the channel tube 117. The insertion portion 2 includes, in the following order from the distal end side, the distal end constituting portion 11, a bending portion 12, and a flexible tube portion 13, which are provided in a linked manner. The image pickup unit and the like are provided in the distal end constituting portion 11.

The bending portion 12 is configured to be bent in two directions, i.e., up and down directions, for example. The operation portion 3 is provided with a bending operation lever 5. The bending portion 12 is configured to be bent in up and down directions by the lever 5 being appropriately operated to allow the bending operation wires (not shown) corresponding to the bending directions to be pulled or relaxed.

Note that the operation portion 3 includes a lever up-direction restricting member 6u and a lever down-direction restricting member 6d. The restricting members 6u, 6d are members for restricting an operation amount of the bending operation lever 5. When the bending operation lever 5 contacts the lever up-direction restricting member 6u, for example, the operation of the lever is stopped. As a result, an active bending portion 14 to be described later, which constitutes the bending portion 12, is prevented from further being bent in the up direction.

In addition, a first index 7a, a second index 7b are provided at predetermined positions at a predetermined interval on the outer surface of the flexible tube portion 13 of the insertion portion 2 so as to be located on the operation portion 3 side. The indices 7a and 7b are notification portions, and notify an operator or the like, whether or not the active bending portion 14 constituting the bending portion 12 of the cholangioscope 1 is placed on the treatment instrument raising stand 120 of the side-view endoscope 101.

Specifically, when only the index 7b can be visually recognized as shown in FIG. 1 in the state where the insertion portion 2 is inserted in the channel tube 117, the index 7b notifies that the active bending portion 14 of the bending portion 12 is positioned on the treatment instrument raising stand 120, as shown in FIG. 3. Such a state where only the index 7b can be visually recognized is defined as a bending operation lever non-operation state.

Such a configuration prevents a problem to occur when the bending direction of a two-direction bending piece group 21, to be described later, of the active bending portion 14 and the pivoting direction of the treatment instrument raising stand 120 do not coincide with each other in the state where the active bending portion 14 is placed on the treatment instrument raising stand 120. If the treatment instrument raising stand 120 is operated to pivot in the state where the bending direction of the two-direction bending piece group 21 and the pivoting direction of the treatment instrument raising stand 120 do not coincide with each other, there is a possibility of occurrence of the problem such as a breakage of the active bending portion 14 or a creation of a hole on the bending rubber.

In other words, when the two indices 7a, 7b can be visually recognized and when the two indices 7a, 7b cannot be visually recognized, the bending operation lever is in an operation state. When the bending operation lever is in the operation state, the insertion portion 2 does not reach the treatment instrument raising stand 120, or a passive bending portion 15 to be described later is placed on the treatment instrument raising stand 120.

Hereinafter, description will be made on the configuration of the bending portion 12 with reference to FIGS. 2 to 4.

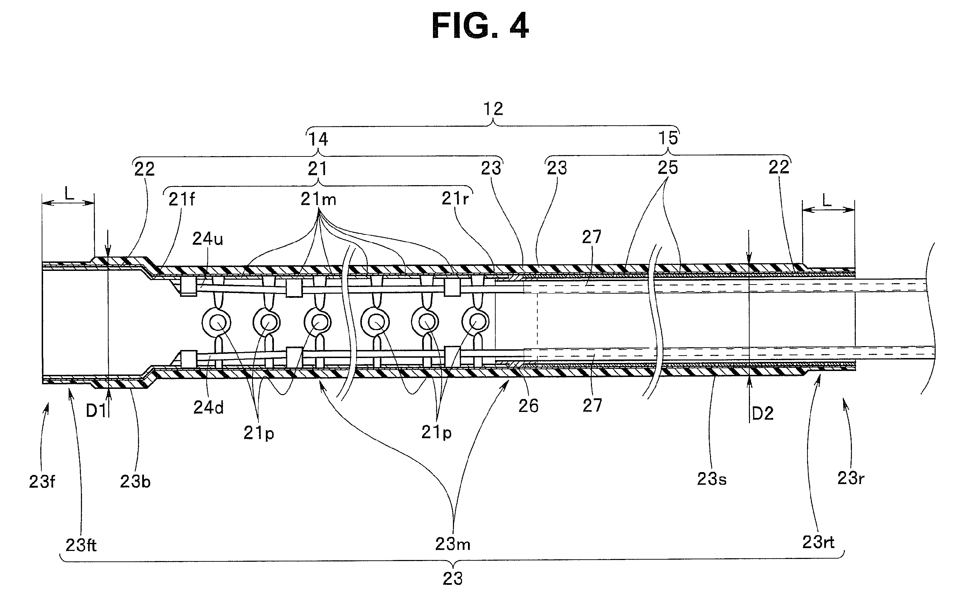

As shown in FIG. 2 and FIG. 4, the bending portion 12 according to the present embodiment includes the active bending portion 14 and the passive bending portion 15 which are provided in a linked manner. The active bending portion 14 configures the distal end constituting portion 11 side of the bending portion 12. The passive bending portion 15 configures the flexible tube portion 13 side of the bending portion 12.

The active bending portion 14 is configured to be bent in two directions, i.e., up and down directions by the bending operation lever 5 being operated. In contrast, the passive bending portion 15 is passively bent by an external force being applied thereto.

The active bending portion 14 mainly includes the two-direction bending piece group 21, a metal mesh tube 22 which covers the bending piece group 21, and a bending rubber 23 as an outer cover. The bending rubber 23 is a tubular flexible member made of elastomer, for example, which has elasticity and biocompatibility.

The two-direction bending piece group 21 includes, in the following order from the distal end side, a distal end bending piece 21f, a plurality of up/down middle bending pieces 21m, and a proximal end bending piece 21r, which are provided in a linked manner. These bending pieces are pivotably supported about an axis with coupling pins 21p. The distal end portions of bending wires 24u, 24d corresponding to the up and down directions are fixed integrally at a predetermined position of the distal end bending piece 21f.

The passive bending portion 15 includes a spiral tube 25, a mesh tube 22 and the bending rubber 23. The spiral tube 25 is made of a thin steel sheet as a core material and formed in a spiral shape. The spiral tube 25 is covered with the mesh tube 22 and the bending rubber 23. That is, the bending rubber 23 configures the outer-most layer of the bending portion 12.

The proximal end bending piece 21r which constitutes the bending piece group 21 and the distal end portion of the spiral tube 25 are integrally bonded to a predetermined fixing portion of the coupling tube 26 by soldering, for example.

In the present embodiment, the mesh tube 22 covers outer circumferences of the two-direction bending piece group 21, the coupling tube 26, and the spiral tube 25. The bending rubber 23 further covers the mesh tube 22 which covers the two-direction bending piece group 21, the coupling tube 26, and the spiral tube 25.

The reference sign 27 represents wire coils. The bending wires 24u and 24d are respectively inserted through the wire coils 27. One end portion of each of the wire coils 27 is fixed to a predetermined position on the inner circumferential surface of the coupling tube 26. The other end portion of each of the wire coils 27 is extended from the proximal end surface of the bending portion 12, passed through the flexible tube portion 13, to be inserted into the operation portion 3.

A distal end portion 23f and a proximal end portion 23r as end portions in the longitudinal axis of the bending rubber 23 as shown in FIG. 4 are provided with an outer-diameter-reduced portion 23ft and a proximal end portion side outer-diameter-reduced portion 23rt. The outer diameter dimensions of the outer-diameter-reduced portion 23ft and the proximal end portion side outer-diameter-reduced portion 23rt are set to be smaller than the outer diameter of a middle tube portion 23m by a predetermined dimension. The outer-diameter-reduced portion 23ft and the proximal end portion side outer-diameter-reduced portion 23rt are outer-diameter-reduced tubular portions whose lengths are set to a predetermined length L in the longitudinal axis direction from the respective end surfaces of the bending portion 12. The outer-diameter-reduced portion 23ft and the proximal end portion side outer-diameter-reduced portion 23rt configure bending portion end small-diameter portions whose outer diameters are smaller than the outer diameter of the part of the bending portion configured by the middle tube portion 23m adjacent to the outer-diameter-reduced portion 23ft and the proximal end portion side outer-diameter-reduced portion 23rt.

In the present embodiment, the thickness of the outer-diameter-reduced portions 23ft, 23rt is formed to be thinner than the thickness of the middle tube portion 23m by a predetermined dimension.

Note that the middle tube portion 23m is provided between the distal end portion side outer-diameter-reduced portion 23ft and the proximal end portion side outer-diameter-reduced portion 23rt.

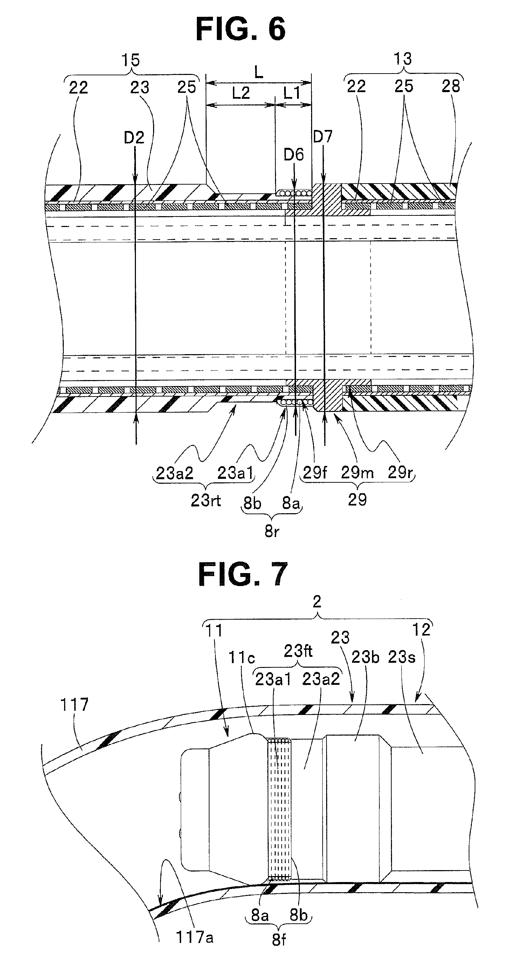

The outer circumferential surface of the distal end portion side outer-diameter-reduced portion 23ft is divided into two areas, that is, a fixing area 23a1 and a non-fixing area 23a2, as shown in FIG. 5. Also the outer circumferential surface of the proximal end portion side outer-diameter-reduced portion 23rt is divided into two areas, that is, a fixing area 23a1 and a non-fixing area 23a2, as shown in FIG. 6.

The fixing areas 23a1 are areas where thread-wound adhering portions 8f, 8r are respectively provided. The fixing areas 23a1 configure end surface sides in the longitudinal axis of the bending rubber 23 and are set so as to have a length L1 from the respective end surfaces.

On the other hand, the non-fixing areas 23a2 are bulged-portion relief portions. The non-fixing areas 23a2 configure middle tube portion end sides in the longitudinal axis and are set so as to have a length L2 from the respective end surfaces of the middle tube portion 23m.

In the present embodiment, the bending rubber 23 has a stepped pipe shape. As shown in FIG. 4, the bending rubber 23 includes, at the middle tube portion 23m, a large-diameter portion 23b with an outer diameter D1 and a small-diameter portion 23s with an outer diameter D2 smaller than the outer diameter D 1.

Note that the shape of the bending rubber 23 is not limited to the stepped pipe shape, but may be a straight pipe shape having a diameter of constant dimension.

Note that the thickness of the middle tube portion 23m is constant.

In the present embodiment, the length of the distal end portion side outer-diameter-reduced portion 23ft and the length of the proximal end portion side outer-diameter-reduced portion 23rt are set to be the same dimension L. However, the length of the distal end portion side outer-diameter-reduced portion 23ft and the length of the proximal end portion side outer-diameter-reduced portion 23rt may be set to different dimensions.

In addition, the length L1 of the fixing areas 23a1 and the length L2 of the non-fixing areas 23a2 are appropriately set in view of the elastic force of the bending rubber 23, the thickness and the length L of the diameter-reduced portions.

With reference to FIGS. 4 and 5, the relation between the bending portion 12 and the distal end constituting portion 11 will be described.

As shown in FIG. 5, the distal end constituting portion 11 includes inside thereof an image pickup optical system 30, an illumination optical system (not shown), a water-feeding channel (not shown), and the like.

The image pickup optical system 30 includes a lens frame 33 having an image pickup window 31 and a plurality of optical lenses 32, a cover glass 34, an image pickup device 35, a substrate 37 on which various kinds of electronic components 36 are mounted, a signal cable 38 that bundles a plurality of signal lines 38a, and the like.

The reference sign 39a represents a first sealing resin, and the reference sign 39b represents a second sealing resin.

The distal end constituting portion 11 is a bending portion fixing rigid portion made of stainless steel, for example. The distal end constituting portion 11 includes in the following order from the distal end side toward the bending portion side end portion: a distal end small-diameter portion 11 a, a first transition portion 11b, a large-diameter portion 11 c, a second transition portion 11d, and a bending portion fixing portion 11e. The distal end portion 23f of the bending portion 12 is fixed to the bending portion fixing portion 11e.

The diameter dimension of the distal end small-diameter portion 11 a is set to be smaller than the large-diameter portion 11c by taking the insertion performance into consideration. The first transition portion 11b forms an inclined surface at which the diameter dimension is continuously changed and increased from the proximal end of the distal end small-diameter portion 11 a toward the distal end of the large-diameter portion 11c. The large-diameter portion 11c is a maximum outer diameter portion of the distal end constituting portion 11 and set to be a predetermined dimension. The second transition portion 11d forms an inclined surface at which the diameter dimension is continuously changed and decreased from the proximal end of the large-diameter portion 11c toward the stepped portion of the bending portion fixing portion 11e.

The bending portion fixing portion 11e is a circumferential stepped portion. On the outer circumferential surface of the circumferential stepped portion, the distal end side inner surface of the distal end bending piece 21f as shown in FIGS. 4 and 5 is arranged. The distal end bending piece 21 f is then integrally bonded on the outer circumferential surface of the bending portion fixing portion 11e by soldering, for example.

The distal end side inner surface of the distal end portion 23f of the bending rubber 23 is disposed at the bending portion fixing portion 11e to which the distal end bending piece 21f is bonded. In this disposed state, mainly the fixing area 23a1 of the distal end portion side outer-diameter-reduced portion 23ft is arranged on the bending portion fixing portion 11e. Then, the distal end side thread-wound adhering portion 8f is provided on the fixing area 23a1 which is the outer circumferential surface of the distal end portion side outer-diameter-reduced portion 23ft.

The distal end side thread-wound adhering portion 8f is a bending portion end fixing portion. The distal end side thread-wound adhering portion 8f includes a tightly bound portion 8a and an adhering portion 8b. The tightly bound portion 8a elastically deforms the distal end portion side outer-diameter-reduced portion 23ft by winding a thread member, to thereby press and fix the distal end portion side outer-diameter-reduced portion 23ft against the step surface of the bending portion fixing portion 11e. The adhering portion 8b is a covering portion that covers and protects the tightly bound portion 8a by applying adhesive to the periphery of the tightly bound portion 8a, to ensure also watertightness.

The outer diameter of the small-diameter portion 11a is D3 and the outer diameter of the large-diameter portion 11c is D4 in the present embodiment. D3 and D4 are set to have the relation of D3 < D4.

In addition, the outer diameter D4 and the outer diameter D1 of the large-diameter portion 23b of the middle tube portion 23m are set to have the relation of D1 ≤ D4.

In addition, in the present embodiment, the outer diameter of the distal end side thread-wound adhering portion 8f is D5, and D5, D4, and D1 are set to have the relation of D5 < D1 ≤ D4.

That is, the outer diameter D5 of the thread-wound adhering portion 8f is smaller than the outer diameter D4 of the large-diameter portion 11c of the distal end constituting portion 11, the large-diameter portion being the bending portion fixing rigid portion-side end portion, and the outer diameter D1 of the distal end portion side outer-diameter-reduced portion side end portion of the large-diameter portion 23b of the bending rubber 23, the large-diameter portion 11c and the distal end portion side outer-diameter-reduced portion side end portion being provided so as to be opposed to each other across the distal end portion side outer-diameter-reduced portion 23ft.

With reference to FIGS. 4 and 6, the relation between the bending portion 12 and the flexible tube portion 13 will be described.

As shown in FIG. 6, the flexible tube portion 13 according to the present embodiment includes a spiral tube 25, a mesh tube 22, and a heat-shrinkable tube 28. The heat-shrinkable tube 28 configures the outer-most layer of the flexible tube portion 13. The heat-shrinkable tube 28 is more rigid than the bending rubber 23 having elasticity. Compared with the flexibility of the bending portion 12, the flexibility of the flexible tube portion 13 is set so as to make the flexible tube portion 13 harder to be bent (more rigid) than the bending portion 12.

The proximal end portion of the passive bending portion 15 which constitutes the bending portion 12 and a distal end portion of the flexible tube portion 13 are coupled and fixed to each other with a connection tube 29.

The connection tube 29 is a bending portion fixing rigid portion made of stainless steel, for example. The connection tube 29 includes in the following order from the distal end side: a distal end side fixing portion 29f; a partition portion 29m, and a proximal end side fixing portion 29r. The proximal end portion 23r of the bending portion 12 is fixed to the distal end side fixing portion 29f.

The distal end side fixing portion 29f and the proximal end side fixing portion 29r are circumferential stepped portions. On the outer circumferential surface of the distal end side fixing portion 29f, the inner circumferential surface of the proximal end portion 23r of the bending portion 12 is arranged. On the proximal end side fixing portion 29r, the inner circumferential surface of the distal end portion of the flexible tube portion 13 is arranged.

In the present embodiment, the proximal end side fixing portion 29r is integrally bonded and fixed, in advance, to the distal end portion of the flexible tube portion 13 by soldering, for example. That is, the flexible tube portion 13 includes the connection tube 29.

The distal end side fixing portion 29f of the connection tube 29 bonded to the distal end portion of the flexible tube portion 13 is disposed on the inner surface of the proximal end portion 23r of the bending portion 12. In this disposed state, mainly the fixing area 23a1 of the proximal end portion side outer-diameter-reduced portion 23rt is arranged on the distal end side fixing portion 29f. The proximal end side thread-wound adhering portion 8r is provided on the fixing area 23a1 which is the outer circumferential surface of the proximal end portion side outer-diameter-reduced portion 23rt.

Also the proximal end side thread-wound adhering portion 8r is a bending portion end fixing portion and includes a tightly bound portion 8a and an adhering portion 8b which covers the tightly bound portion 8a to ensure watertightness. The tightly bound portion 8a elastically deforms the proximal end portion side outer-diameter-reduced portion 23rt to press and fix the proximal end portion side outer-diameter-reduced portion 23rt against the stepped surface of the distal end side fixing portion 29f.

In the present embodiment, the outer diameter of the proximal end side thread-wound adhering portion 8r is D6, and the outer diameter of the partition portion 29m of the connection tube 29 is D7.

D2 and D6 are set to have the relation of D6 < D2.

In addition, D7, D6, and D2 are set to have the relation of D6 < D2 ≤ D7.

That is, the outer diameter D6 of the proximal end side thread-wound adhering portion 8r is smaller than the outer diameter D7 of the partition portion 29m of the connection tube 29, the partition portion 29m being the bending portion fixing rigid portion-side end portion, and the outer diameter D2 of the proximal end portion side outer-diameter-reduced portion side end portion of the small-diameter portion 23s of the bending rubber 23, the partition portion 29m and the proximal end portion side outer-diameter-reduced portion side end portion being arranged so as to be opposed to each other across the proximal end portion side outer-diameter-reduced portion 23rt.

Note that the outer diameters D5, D6 of the above-described thread-wound adhering portions 8f, 8r are set in view of the outer diameters of the stepped outer circumferential surfaces of the circumferential stepped portions, the diameter of the thread that constitutes the thread-wound adhering portion 8, and the thickness of the adhering portion when the adhesive is hardened.

Now, description will be made on the working of the cholangioscope 1 configured as described above.

When performing examination of the bile duct, the operator inserts the second distal end portion 111 of the second insertion portion 102 of the second endoscope 101 into the duodenum 130 and places the second distal end portion 111 in the duodenum 130.

Next, the operator inserts the insertion portion 2 of the cholangioscope 1 into the channel tube 117 through the treatment instrument insertion port 118 of the second endoscope 101. Then, the operator allows the insertion portion 2 to pass through the channel tube 117 and leads out the insertion portion 2 from the distal end opening 116, and thereafter allows the insertion portion 2 to pass over the treatment instrument raising stand 120 and leads the insertion portion 2 to the outside from the outer circumferential side surface 114 of the second distal end portion 111, while observing the endoscopic image.

Next, after the operator confirms that the second index 7b has been inserted into the channel tube 117, in other words, when the two indices 7a and 7b are not visually confirmable, the operator operates the treatment instrument raising stand operation lever 121 to cause the treatment instrument raising stand 120 to pivot.

In accordance with the pivoting of the treatment instrument raising stand 120, the leading-out direction of the insertion portion 2 led out from the outer circumferential side surface 114 is adjusted. As a result, the insertion portion 2 is selectively inserted into the bile duct 131, for example. Note that the reference sign 132 represents a pancreatic duct.

After the completion of the examination, the cholangioscope 1 is extracted through the channel tube 117.

As described above, the outer diameter of the distal end side thread-wound adhering portion 8f is smaller than the outer diameter of the large-diameter portion 11c and the outer diameter of the outer-diameter-reduced portion side end portion of the large-diameter portion 23b of the bending rubber 23, the large-diameter portion 11c and the outer-diameter-reduced portion side end portion of the large-diameter portion 23b being arranged so as to be opposed to each other across the distal end portion side outer-diameter-reduced portion 23ft. In addition, as shown in FIG. 6, also in the proximal end side thread-wound adhering portion 8r, the outer diameter of the thread-wound adhering portion 8r is smaller than the outer diameter portion of the partition portion 29m and the outer diameter of the outer-diameter-reduced portion side end portion of the small-diameter portion 23s of the bending rubber 23, the partition portion 29m and the outer-diameter-reduced portion side end portion of the small-diameter portion 23s being opposed to each other across the proximal end portion side outer-diameter-reduced portion 23rt.

As a result, the working to be described below can be obtained.

As shown in FIG. 7, when the distal end portion side of the insertion portion 2 passes through the channel tube 117, for example, the distal end side thread-wound adhering portion 8f is prevented from contacting the inner surface 117a. This is because the large-diameter portion 11c and the outer-diameter-reduced portion side end portion of the large-diameter portion 23b of the bending rubber 23, which are provided so as to be opposed to each other across the distal end portion side outer-diameter-reduced portion 23ft, contact the inner surface 117a of the channel tube.

Similarly, the proximal end side thread-wound adhering portion 8r is prevented from contacting the inner surface 117a.

Furthermore, when the insertion portion 2 passes over the treatment instrument raising stand 120, the thread-wound adhering portions 8f, 8r are prevented from contacting the treatment instrument arranging surface (see the reference sign 124 in FIG. 3).

Note that illustration of the proximal end side thread-wound adhering portion 8r is omitted in FIG. 7.

In addition, each of the outer-diameter reduced portions 23ft, 23rt is divided into two areas, i.e., the fixing area 23a1 and the non-fixing area 23a2. Each of the fixing areas 23a1 is set on the longitudinal axis end surface side, and provided with each of the thread-wound adhering portions 8f and 8r. As a result, the working to be described below can be obtained.

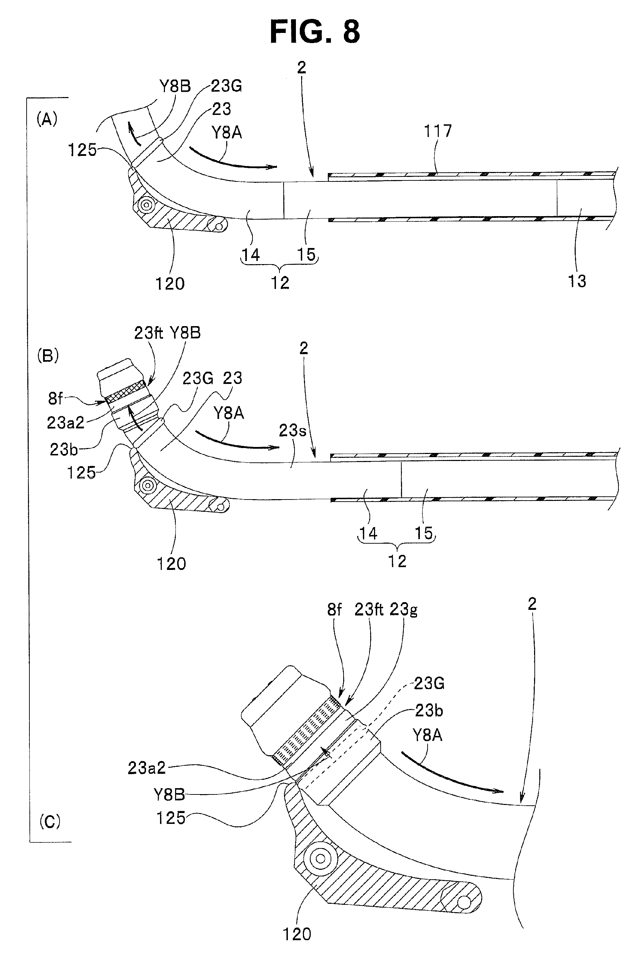

For example, when the insertion portion 2 is extracted after the completion of the examination, friction is generated between a stand distal end portion 125 of the treatment instrument raising stand 120 and the surface of the bending rubber 23. This friction then causes slackening on the surface of the bending rubber 23, and the slackening becomes folds. The folds deform to be a bulged portion 23G as shown in FIG. 8 (A) and such a bulged portion sometimes appears in the vicinity of the stand distal end portion 125.

The appeared bulged portion 23G is moved in accordance with the extraction of the insertion portion 2 in the direction of the arrow Y8B, which is opposite direction of the arrow Y8A as the insertion portion extraction direction, due to the elasticity of the bending rubber 23. Then, as shown in FIG. 8 (B), the bulged portion 23G moves near to the large-diameter portion 23b.

After that, as the insertion portion 2 is further extracted, the bulged portion 23G reaches the large-diameter portion 23b as shown by the dashed lines in FIG. 8(C), and thereafter passes through the large-diameter portion 23b to reach the non-fixing area 23a2 of the distal end portion side outer-diameter-reduced portion 23ft.

Then, the bulging amount of the bulged portion 23G decreases in accordance with the thickness and elastic force of the bending rubber, and the bulged portion 23G changes to a reduced bulged portion 23g, the outer diameter of which is equal to or smaller than D4 and D1. As a result, the reduced bulged portion 23g is not likely to be rubbed by the stand distal end portion 125 of the treatment instrument raising stand 120. Therefore, this enables the reduced bulged portion 23g to pass over the raising stand 120, which reduces the occurrence frequency of creation of holes.

The outer-diameter-reduced portions 23ft and 23rt, the thickness of which is set to be thinner than the thickness of the middle tube portion 23m, are provided on the both end portions of the bending rubber 23 having elasticity. Then, each of the outer-diameter-reduced portions 23ft, 23rt is divided into two areas, that is, the fixing area 23a1 which constitutes the end surface side, and the non-fixing area 23a2 which is adjacent to the fixing area 23a1. Each of the thread-wound adhering portions 8f and 8r is provided on the fixing area 23a1. Then, the outer diameters of the thread-wound adhering portions 8f, 8r are set to be smaller than the outer diameters of the bending portion fixing rigid portion end portion and the end portion of the middle tube portion which are arranged so as to be opposed to each other across the outer-diameter-reduced portions 23ft and 23r.

As a result, it is possible to prevent the thread-wound adhering portions 8f and 8r from contacting the inner surface of the tube or the arranging surface of the raising stand, when the insertion portion 2 is inserted and extracted through the channel tube 117 and the treatment instrument raising stand 120. This prevents occurrence of such problems that the adhering portions 8b of the thread-wound adhering portions 8f and 8r are peeled off and the thread of the tightly bound portions 8a of the thread-wound adhering portions 8f and 8r are cut off.

In addition, when the bulged portion 23G appears due to friction, the bulged portion 23G reaches the non-fixing areas 23a2 of the outer-diameter-reduced portions 23ft, 23rt, to be changed to the reduced bulged portion 23g formed by the bulging amount of the bulged portion 23G being decreased. As a result, creation of holes due to the appearance of the bulged portion 23G can be prevented.

Furthermore, at the time of replacement of the bending rubber 23, it is possible to easily detach the bending rubber 23 by breaking the adhering portions 8b of the thread-wound adhering portions 8f and 8r provided on the fixing areas 23a1 of the outer-diameter-reduced portions 23ft and 23rt, and thereafter cutting the tightly bound portions 8a. Therefore, repairability of the bending rubber can be maintained.

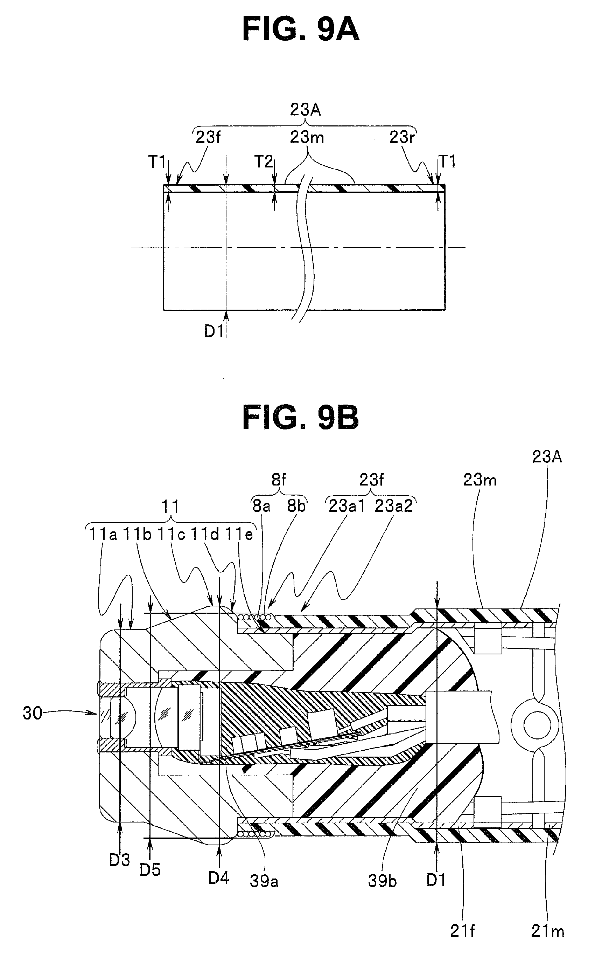

Note that the bending rubber 23 according to the above-described embodiment includes, at the respective end portions thereof, the outer-diameter small-diameter portions 23ft, 23rt whose thickness is formed to be thinner than the thickness of the middle tube portion 23m by a predetermined dimension. However, as shown in FIG. 9A, a thickness T1 of a distal end portion 23f and a proximal end portion 23r of a bending rubber 23A may be equal to a thickness T2 of a middle tube portion 23m, that is, the thickness of the bending rubber may be constant.

In this configuration, a piece small-diameter portion 21fs at which the end portion of the bending rubber 23A is arranged is formed at the distal end bending piece 21f, as shown in FIG. 9B. Then, the distal end portion of the bending rubber 23 is arranged at the piece small-diameter portion 21fs of the distal end bending piece 21f. As a result, the bending portion end small-diameter portion is formed on the distal end side of the bending portion 12 in the present embodiment.

Note that a piece small-diameter portion 21rs is formed on the proximal end bending piece 21r, though illustration thereof is omitted. The proximal end portion of the bending rubber 23A is arranged at the piece small-diameter portion 21rs of the proximal end bending piece 21r. As a result, on the proximal end side of the bending portion 12, the proximal end side bending portion end small-diameter portion is formed.

Consequently, as described above, in the relation in which the thickness of the distal end portion 23f and proximal end portion 23r of the bending rubber 23A is equal to the thickness of the middle tube portion 23m, the outer diameter D5 of the thread-wound adhering portion 8f provided on the fixing area 23a1 and the outer diameter of the non-fixing area 23a2 provided at the piece small-diameter portion 21fs are smaller than the outer diameter D4 of the large-diameter portion 11c of the distal end constituting portion 11 and the outer diameter D1 of the end portion of the bending rubber 23, the large-diameter portion 11c and the end portion of the bending rubber being arranged so as to be opposed to each other across the bending portion end small-diameter portion.

Therefore, as described above, it is possible to prevent occurrence of such problems that the adhering portions 8b are peeled off and the threads of the tightly bound portions 8a are cut off, which are caused by the contact between the thread-wound adhering portions 8f, 8r of the bending rubber 23A and the inner surface of the tube or the arranging surface of the raising stand. Furthermore, when the bulged portion 23G appears due to friction, the bulged portion 23G reaches the non-fixing area 23a2 provided at the piece small-diameter portion 21fs to be changed to the reduced bulged portion 23g formed by the bulging amount of the bulged portion 23G being reduced, which prevents the creation of holes, and the like.

In addition, similarly as described above, the adhering portions 8b of the thread-wound adhering portions 8f, 8r provided on the end portions of the bending rubber 23A are broken, and then the tightly bound portions 8a are cut, thereby enabling the bending rubber 23 to be easily detached. Therefore, it is possible to maintain the repairability of the bending rubber.

In addition, in the above-described embodiment, the lengths of the active bending portion 14 and passive bending portion 15 which constitute the bending portion 12 are appropriately set, to allow the passive bending portion 15 to be arranged on the distal end side of the channel tube 117 when the distal end constituting portion 11 of the insertion portion 2 reaches a predetermined site of the bile duct 131 or the pancreatic duct 132.

According to this configuration, the flexible tube portion 13 reaches the arranging surface of the treatment instrument raising stand 120 to be raised, thereby capable of preventing wrinkles from being created on the heat-shrinkable tube 28.

Note that, when the treatment instrument raising stand 120 is raised, wrinkles are created on the bending rubber 23. However, when the raising stand 120 is returned to the original state, the wrinkles created by the elastic force of the bending rubber 23 are erased.

In order to prevent the passive bending portion 15 covered with the bending rubber 23 from buckling while in use, the strength of the spiral tube 25 constituting the passive bending portion 15 is more increased than that of the spiral tube 25 constituting the flexible tube portion 13, or the strength of the mesh tube 22 constituting the passive bending portion 15 is more increased than that of the mesh tube 22 constituting the flexible tube portion 13.

Specifically, when the strength of the spiral tube 25 constituting the passive bending portion 15 is set to be higher, the width of the spiral tube 25 constituting the passive bending portion 15 is set to be wider than the width of the spiral tube 25 constituting the flexible tube portion 13.

On the other hand, when the strength of the mesh tube 22 constituting the passive bending portion 15 is set to be higher than the strength of the mesh tube 22 constituting the flexible tube portion 13, density, wire cross-sectional shape, or wire rigidity of the mesh tube 22 constituting the passive bending portion 15 is changed, for example. Specifically, the density of the mesh tube 22 constituting the passive bending portion 15 is set to be higher than the density of the mesh tube 22 constituting the flexible tube portion 13. Alternatively, the wire cross-sectional shape of the mesh tube 22 constituting the passive bending portion 15 is made to be flatter than the wire cross-sectional shape of the mesh tube 22 constituting the flexible tube portion 13. For example, when the wire cross-sectional shape of the mesh tube 22 constituting the passive bending portion 15 is circular shape, the wire cross-sectional shape of the mesh tube 22 constituting the flexible tube portion 13 is made to be rectangular shape, or the like, or the rigidity of the wire of the mesh tube 22 constituting the passive bending portion 15 is made to be higher than the rigidity of the wire of the mesh tube 22 constituting the flexible tube portion 13.

Note that the bending portion 12 is configured to include the active bending portion 14 and the passive bending portion 15 which are provided in a linked manner in the above-described embodiment. However, the bending portion 12 may be provided only with the active bending portion 14. In that case, the bending rubber 23 configured as described above covers the active bending portion 14, and the proximal end bending piece 21r constituting the active bending portion 14 is coupled and fixed to the flexible tube portion 13 through the connection tube 29.

When the cholangioscope 1 configured to be bent in two directions is inserted into the channel tube 117 of the side-view endoscope 101 including the treatment instrument raising stand which is configured to pivot, matching the up/down direction of the cholongioscope 1 with the up/down direction of the side-view endoscope 101 enables smooth insertion of the cholangioscope 1. However, as the insertion portion 2 advances in the channel tube 117, gradual misalignment is likely to occur between the up/down direction of the cholangioscope 1 and the up/down direction of the side-view endoscope 101. Increase in the amount of misalignment may cause a problem of an increase in insertion force amount.

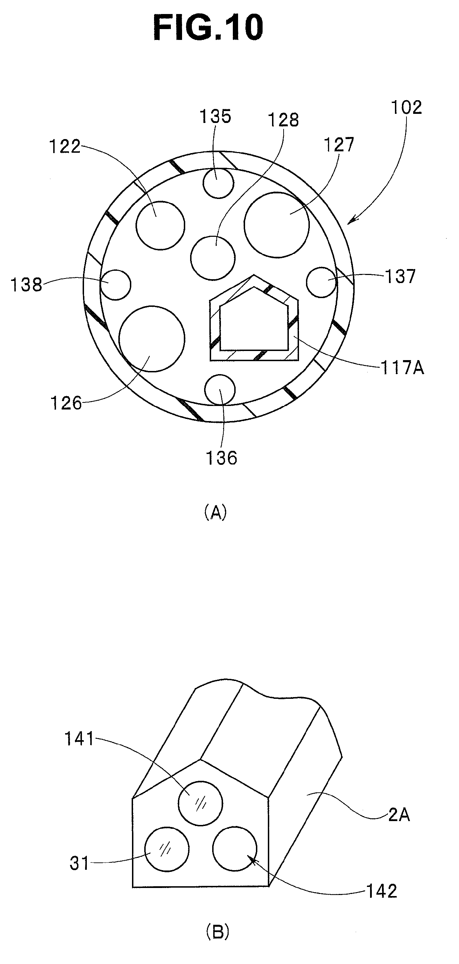

In order to eliminate this problem, as shown FIG. 10(A), the cross-sectional shape of the inner surface of the channel tube 117A inserted and arranged in the second insertion portion 102 is configured to be non-circular shape, for example, pentagonal shape. On the other hand, as shown in FIG. 10(B), the cross-sectional shape of the insertion portion 2A of the cholangioscope 1 is set to be pentagonal shape in accordance with the cross-sectional shape of the inner surface of the channel tube 117A.

According to this configuration, it is possible to surely prevent the misalignment between the up/down direction of the cholangioscope 1 and the up/down direction of the side-view endoscope 101 which occurs as the insertion portion 2A of the cholangioscope 1 advances in the channel tube 117A. As a result, the configuration is capable of providing the smooth insertability of the insertion portion 2A of the cholangioscope 1 into the channel tube 117A.

Note that the reference sign 126 represents a second light guide fiber bundle which constitutes the illumination optical system, the reference sign 127 represents a second signal cable which extends from the image pickup unit, the reference sign 128 represents an air/water feeding tube, the reference sign 135 represents a second up direction bending wire, the reference sign 136 represents a second down direction bending wire, the reference sign 137 represents a right direction bending wire, the reference sign 138 represents a left direction bending wire, the reference sign 141 represents the illumination window, and the reference sign 142 represents a fluid port which also serves as a treatment instrument opening.

When the insertion portion 2 of the cholangioscope 1 is inserted into the channel tube 117 with the guide wire retained, it is necessary to pass the guide wire through the channel of the cholangioscope 1. However, at this time, the operator has to hold the guide wire to prevent the guide wire from being unintentionally inserted into the deep part of the bile duct. Therefore, the guide wire is passed through the channel tube 117 of the second endoscope 101 to be led to the outside, and then further passed through the channel of the cholangioscope 1 to be led to the outside. As a result, the length of the guide wire becomes four meters or longer.

The operation for guiding the insertion portion 2 of the cholangioscope 1 to a target site to be observed through the guide wire of four meters or longer has been a troublesome procedure, since handling of the guide wire is complicated.

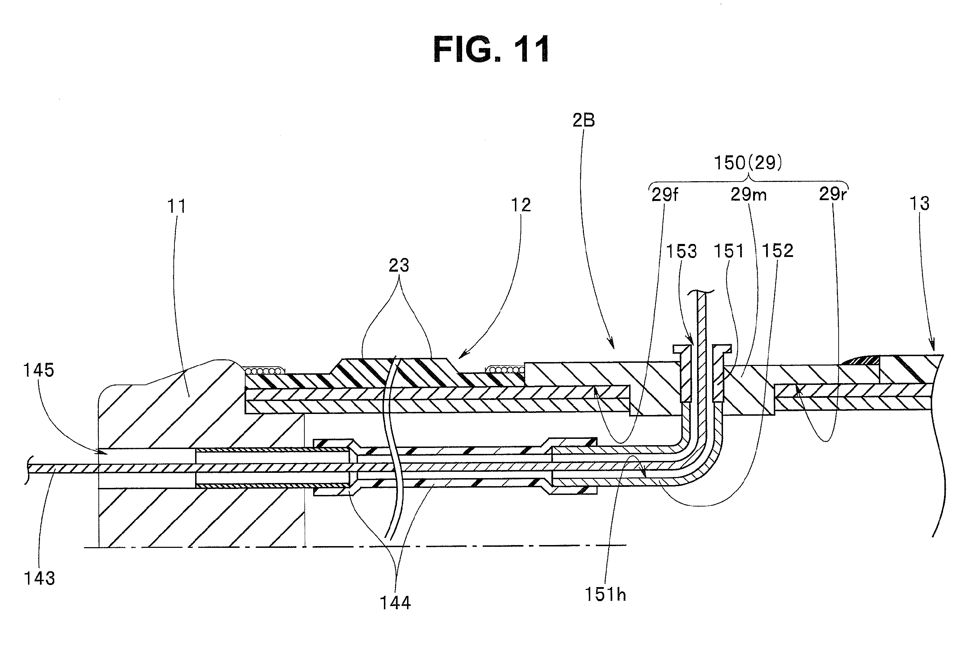

In order to solve this trouble, a guide-wire-dedicated channel tube (hereinafter, shortly referred to as GW tube) 144 through which the guide wire 143 is inserted is provided in the insertion portion 2B as shown in FIG. 11.

The distal end portion of the GW tube 144 is communicated with a GW opening 145 which serves also as the treatment instrument opening of the distal end constituting portion 11, to be fixed to the GW opening. The proximal end portion of the GW tube 144 is communicated with a through hole 151h of a GW insertion member 151 provided to a ring for guide wire (hereinafter, shortly referred to as GW ring) 150, to be fixed to the through hole 151h.

The GW ring 150 serves also as the above-described connection tube 29, for example. The GW ring 150 includes, at the partition portion 29m, the GW insertion member 151 including a tube fixing pipe 152 provided integrally with the GW insertion member. The GW insertion member 151 is fixed to the GW ring 150 in a watertight manner. The GW tube 144 is provided in the insertion portion 2B of the cholangioscope 1, and the end portion of the GW tube 144 is communicated with the through hole 151h of the GW insertion member 151 provided at the halfway position of the insertion portion, the halfway position being apart from the distal end of the insertion portion 2B by a predetermined distance.

As a result, the length of the guide wire 143 is set to a predetermined length according to the length enough for the guide wire to be passed through inside of the channel tube 117 of the second endoscope 101 and led out to the outside. In other words, the length of the guide wire 143 is set to be shorter than four meters.

When guiding the insertion portion 2B of the cholangioscope 1 to a target site to be observed through the guide wire 143, the operator first inserts the guide wire 143 extended from the treatment instrument insertion port 118 of the second endoscope 101 from the GW opening 145 of the cholangioscope 1, to lead the guide wire out from a guide wire leading-out port 153.

Next, the operator inserts the insertion portion 2B of the cholangioscope 1 from the treatment instrument insertion port 118 into the channel tube 117 while holding the led-out guide wire 143. After that, the operator gradually inserts the insertion portion 2B into the deep part of the channel tube 117 by using the wire 143 as a guide.

Thus, the GW tube 144 into which the guide wire 143 is inserted is provided in the insertion portion 2B of the cholangioscope 1. In addition, the guide wire leading-out port 153 is provided at the halfway position of the insertion portion, the halfway position being away from the distal end of the insertion portion 2B by a predetermined distance. As a result, the handling of the guide wire 143 becomes easy when the insertion portion 2B of the cholangioscope 1 is guided to the target site to be observed through the guide wire 143, which eliminates the complicated handling of the guide wire.

Note that the present invention is not limited only to the above-described embodiments and various modifications are possible in a range without departing from the gist of the invention.

This application claims the benefit of Japanese Application No. 2013-128837 filed in Japan on June 19, 2013, and the above described disclosure is incorporated by reference in the present description, claims, and drawings.

## Claims

1. An endoscope comprising: a tubular flexible member which has elasticity and configures an outermost layer of a bending portion; a bending portion fixing rigid portion at which an end portion of the tubular flexible member is arranged; and a bending portion end fixing portion for fixing the end portion of the tubular flexible member arranged at the bending portion fixing rigid portion,
wherein the bending portion includes, at an end portion in a longitudinal axis thereof, a bending portion end small-diameter portion, an outer diameter dimension of which is set to be small, and
an outer diameter of a covering portion which configures the bending portion end fixing portion is smaller than an outer diameter of the bending portion fixing rigid portion-side end portion and an outer diameter of an end portion of a middle tube portion of the tubular flexible member, the bending portion fixing rigid portion-side end portion and the end portion of the middle tube portion being arranged so as to be opposed to each other across the bending portion end small-diameter portion.

2. The endoscope according to claim 1, wherein the bending portion end small-diameter portion is configured such that a thickness of the end portion of the tubular flexible member is set to be thinner than a thickness of the middle tube portion.

3. The endoscope according to claim 1, wherein the bending portion end small-diameter portion is configured by arranging the end portion having a constant thickness of the tubular flexible member at a bending piece small-diameter portion formed at a distal end bending piece and at a bending piece small-diameter portion formed at a proximal end bending piece.

4. The endoscope according to claim 2 or 3, wherein
an outer circumferential surface of the bending portion end small-diameter portion is divided into a fixing area which configures an end surface side in the longitudinal axis and a non-fixing area which configures a middle tube portion side in the longitudinal axis, and
the bending portion end fixing portion is a thread-wound adhering portion provided on the fixing area, the thread-wound adhering portion including: a thread member which tightly binds the bending portion end small-diameter portion; and the covering portion which is configured by applying adhesive so as to cover a portion tightly bound by the thread member.

5. The endoscope according to claim 4, wherein an outer diameter dimension of the bending portion fixing rigid portion-side end portion is equal to or larger than an outer diameter dimension of the middle tube portion of the tubular flexible member.

6. The endoscope according to claim 1, wherein the bending portion fixing rigid portion is made of metal or resin, a rigidity of which is higher than rigidities of the tubular flexible member and the bending portion end fixing portion.
